# EUROPEAN PATENT APPLICATION

(11) **EP 1 739 080 A1**
(43) Date of publication of application: **03.01.2007**
(21) Application number: 06115619.6
(22) Date of filing: 19.06.2006
(51) Int. Cl.: C07D 207/46, G01N 33/68

(54) **Ionization Modifier for Mass Spectrometry**

(30) Priority: 30.06.2005 EP 05105955
(71) Applicant: F. HOFFMANN-LA ROCHE AG, 4070 Basel (CH)
(72) Inventor: Doebeli, Heinz, 4417, Ziefen (CH)

(57) **Abstract**

The present invention is concerned with a compounds of the general formula 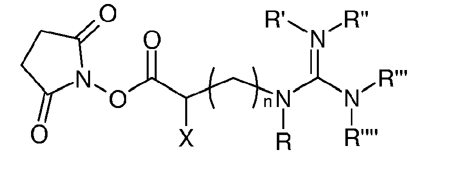 wherein R, R', R", R"', R"", X and n arc as defined in the description and the claims and the use of said compound as ionization modifier. The invention further relates to a method for the quantification of a polypeptide or fragments thereof from a source containing said polypeptide in labeled and unlabeled form comprising (a) modifying the isolated polypeptide with a ionization modifier, (b) analyzing the prepared polypeptide or fragments thereof by mass spectrometry, and thereby determining the amount of polypeptide or fragments thereof that was present in the source of polypeptide or fragments thereof.

## Description

Mass spectrometry has evolved to a versatile technique in analytical protein biochemistry. The instrument type of choice are MALDI―TOF mass spectrometers and the analytical window is optimized for the type of peptides with molecular masses between 0.8 and 3 kDa generated by cleavage with endopeptidases as trypsin or endoproteinase-C.

However, despite the fact that the cleavage procedure is designed to generate peptides with a homogeneous distribution of basic residues which warrant ionization, some molecules are detected more readily than others. This is due to ionization competition which may result in complete quenching of some peptides. A prominent example is the Alzheimer's amyloid peptide (Aβ). It is known, that the three peptide fragments of Aβ generated with the endopeptidase Lys-C behave very differently (Grüninger at al. 2000, Identification of beta-secretase-like activity using a mass spectrometry-based assay system. Nature Biotechnology, 18:66-70). The amino-terminal fragment and the middle fragment have excellent ionization properties whereas the carboxy-terminal fragment has never been detected using the preferred MALDI -TOF method (Rüfenacht et al. 2005, Quantification of the Aβ peptide in Alzheimer's plaques by laser dissection microscopy combined with mass spectrometry. J. Mass Spectrom.; 40:193-201).

But the carboxy-terminus is considered to be the crucial part of the amyloid peptide. Aβ occurs in different chain length. The majority of the peptides end at residues 40, whereas a small amount ends at residue 42. The longer version has a much higher propensity to form fibers and is thus considered to be the culprit.

Furthermore, it would be of interest to differentiate the quantities of different Abeta species caused by modifications such as the Aβ(11-16) and Aβ(pyroGlu11-16) fragments (Huse et al., 2002, γ-Secretase processing in the trans-Golgi network preferentially generates truncated amyloid species that accumulate in Alzheimer's disease brain. J. Biol Chem. 277:16278-16284) or Abeta with methionine sulfoxide at position 35 in addition to its natural form as methionine. Hou et al (Methionine-35 oxidation reduces fibril assembly of the amyloid Ab-(1-42) peptide of Alzheimer's disease. J. Biol. Chem.(2002) 277:40172-40176) showed that the oxidation of the Met-35 side chain to a methionine sulfoxide (Met-35(ox)) significantly hinders the rate of fibril formation for the 42-residue Abeta-KM/10.04.2006 (1-42) at physiological pH. Met-35(ox) also alters the characteristic Abeta fibril morphology and prevents formation of the protofibril, which is a key intermediate in beta-amyloidosis and the associated neurotoxicity.

Thus, there is a strong need for a method to detect such molecules previously escaping the detection by mass spectrometry.

Therefore, the present invention relates to novel molecules which modify polypeptides and thereby allow the detection of polypeptides with mass spectrometry which previously escaped the detection by this method.

The present invention provides a compound of the general formula: 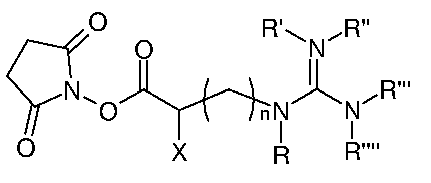
- n =: 0,1,2,3,4,5, 6, 7 or 8;
- X =: H, OH, F, Cl, Br, CH₃, C₂H₅, C₃H₇ or C₄H₉;
- R =: no residue, H, CH₃, C₂H₅, C₃H₇ or C₄H₉;
- R' =: no residue, H, CH₃, C₂H₅, C₃H₇ or C₄H₉;
- R" =: H, CH₃, C₂H₅, C₃H₇ or C₄H₉;
- R''' =: H, CH₃, C₂H₅, C₃H₇ or C₄H₉;
- R'''' =: H, CH₃, C₂H₅, C₃H₇ or C₄H₉;
or R' and R" or R" and R''' or R''' and R"" or R and R"" are bonded to each other to form a ring together with the nitrogen atoms to which they are attached, and R' and R" or R" and R''' or R''' and R"" or R and R"" together are:

-CH₂-(CH₂)ₚ-,

wherein p is 1, 2 or 3;
or X and R are bonded to each other to form a ring together with the nitrogen atom and carbon atom respectively to which they are attached, and X and R together are:

- (CH₂)ₖ-,

wherein k is 1, 2, 3 or 4.

Preferably, n = 1, X = OH or H, R = H, R' = no residue, R" = H, R''' = H and R"" = H. Also preferred is the compound as described above wherein n = 4, X = H, R = H, R' = no residue, R" = H, R''' = H and R"" = H.

X determines the reactivity of the conversion reaction to yield the amide bond between analyte and ionization modifier. When X is Cl or Br, it is possible to identify the modified amine in a complex peak pattern. Chlorine or bromine contained in the ionization modifier are present as a mixture of isotopes and generate a distinct pattern of double bands. When analytes are modified with such a label, the corresponding adduct is easily identified among other peaks by the peak pattern.

The invention further provides the use of the compounds described above as ionization modifier.

The term "ionization modifier" or "flight modifier" as used herein refers to a molecule which is capable of binding to the polypeptide of interest and by binding modifying the behaviour of the polypeptide of interest in the mass spectrometry. The ionization modifier is able to accept or donate at least one electron. Preferably, the ionization modifier is specific for one amino acid (i.e. Lysine).

The term "polypeptide" or "polypeptide of interest" as used herein refers to amino acid chains of various length. A polypeptide may be i.e. a protein or fragments thereof, or a peptide or fragments thereof. Preferably, the polypeptide is a C-terminal fragment of beta-amyloid peptide Abeta 1-40 and Abeta 1-42, such as e.g. Abeta(29-40) and Abeta(29-42).

Furthermore, the present invention provides a method for the quantification of a polypeptide of interest from a source comprising said polypeptide in labeled and unlabeled form, said method comprising the following steps:
(a) modifying the isolated polypeptide with a ionization modifier,
(b) analyzing the prepared polypeptide by mass spectrometry, and thereby determining the amount of polypeptide of interest that was present in the source of the polypeptide.

One embodiment of the present invention relates to a method for the quantification of a polypeptide of interest comprising
(a) providing a source of the polypeptide,
(b) adding a defined amount of the polypeptide labeled with a stable isotope to the source of (a),
(c) isolating unlabeled and labeled polypeptide of interest,
(d) preparing the isolated polypeptide of interest for analysis by mass spectrometry,
(e) modifying the isolated polypeptide with a ionization modifier,
(f) analyzing the prepared polypeptide of interest by mass spectrometry, and
(g) determining the amount of polypeptide of interest that was present in the source of polypeptide.

The source of the polypeptide of interest may be body fluid as i.e. blood, or the polypeptide of interest may be obtained from tissue samples (e.g. homogenized brain samples) or cell cultures. The tissue samples, body fluid or cell cultures may be a mammalian tissue sample, mammalian body fluid or mammalian cells. Preferred tissue samples, cells and body fluids derived from human or mouse.

In the sources, the polypeptide of interest may be present in soluble or aggregated form. The aggregated polypeptide of interest may form plaques whereby the term "plaque" as used herein refers to a deposit of aggregated polypeptides. Sources of the polypeptide of interest containing aggregated polypeptide (i.e. amyloid deposits containing aggregated beta amyloid) may be obtained from tissue samples by methods comprising general biochemical polypeptide purification methods and methods for specific excision of structures from tissues comprising laser dissection microscopy.

The laser dissection microscopy method comprises the steps of cold ablation and laser pressure catapulting (Schütze et al (1998), Identification of expressed genes by laser-mediated manipulation of single cells, Nature Biotechnology 16: 737-742; Simone et al (1998), Laser-capture microdissection: opening the microscopic frontier to molecular analysis, TIG 14: 272-276). Laser dissection microscopy can be used to capture any specific phenotypes or phenotypic tissue changes identifiable by light microscopy. As an example, this technique could help in detecting differences in gene expression between normal cells or tissues and pathological material by separate microdissection and analysis (e.g. by microarray) of the isolated specimen. Qualitative and quantitative analysis of critical changes thus can be performed more easily and with more accuracy compared to the analysis of whole tissues as is necessary without laser dissection. The advantages of isolating structures of interest by laser dissection prior to analyzing the polypeptide compositions is useful, where not average polypeptide compositions or concentrations are needed, but where specific biological structures need to be analyzed.

The excised source containing aggregated polypeptide of interest may only represent a fraction of the whole plaque. In case of Abeta the plaque is spherical. To determine the amount of polypeptide of interest in the whole plaque, the amount of polypeptide of interest determined in the excised part of the plaque has to be balanced by a correction factor. The correction factor depends from the thickness of the tissue slice and the average plaque diameter and extrapolates the amyloid content of the excised disc to the entire sphere (Andreas Güntert "laser dissection microscopy in the comparison of plaques from human and transgenic mice" Diploma thesis 2002, Biozentrum der Universität Basel, Switzerland).

Furthermore, after excision, the plaques may be transferred to a vessel by an electrostatic effect.

The presence of the polypeptide of interest in the tissue sample, cell cultures or body fluid may be determined by methods comprising polypeptide biochemistry, histochemistry and immunochemistry. Aggregated polypeptide of interest may preferably determined in a tissue sample histochemical methods comprising staining with Congo Red or Thioflavin S, or by immunohistochemical methods. More preferably, the presence of the aggregated polypeptide of interest in a tissue sample is determined by double staining with histochemical and immunohistochemical methods. Such methods are well known to the skilled person in the art. Most preferably, the presence of the aggregated polypeptide of interest in a tissue sample is determined by staining first with Congo Red followed by immunohistochemistry. Preferably, the presence of polypeptide of interest in body fluid is determined by Western blotting of a body fluid sample.

In the methods of the present invention, the polypeptide of interest labeled with a stable isotope is added as a standard to the source of polypeptide of interest before the start of the dissolution and/or isolation procedure. This standard, the polypeptide of interest labeled with a stable isotope, is added directly to the homogenized tissue sample, the excised polypeptide deposit, the body fluid sample or the cell culture.

The standard (polypeptide of interest labeled with a stable isotope) can be spiked at the very beginning into the source of polypeptide of interest, e.g., into excised polypeptide deposits or into a sample of body fluid. As the unlabeled polypeptide of interest to be quantified and the labeled polypeptide of interest standard are chemically identical except for mass difference in identical atoms, they behave identically in the required dissolution and/or isolation procedure of the aggregated polypeptide of interest or of soluble polypeptide of interest (e.g. aggregated amyloid or soluble amyloid) which may be bound by other polypeptides, which results in equal losses of the analyte and the standard. A comparison of the amount of labeled standard before and after the procedure allows the determination of the amount of unlabelled polypeptide of interest originally present in the source.

The polypeptide of interest standard is labeled with at least one stable isotope selected from the group comprising ²H, ¹³C, ¹⁵N, and ¹⁸O. Preferably, the polypeptide of interest standard is labeled with ¹⁵N or ¹³C. More preferably, the polypeptide of interest standard is labeled with ¹⁵N. Preferably, the polypeptide of interest standard is labeled with as many stable isotopes as necessary for the separation of the isotope patterns in the mass spectra.

The polypeptide of interest standard labeled with a stable isotope is added in a defined amount. Preferably, the labeled polypeptide of interest standard is added in an amount in the same range as the effective amount of polypeptide of interest present in the source of polypeptide of interest. This amount maybe determined in preliminary experiments, e.g. as described in Rüfenacht et al. (Quantification of the Aβ peptide in Alzheimer's plaques by laser dissection microscopy combined with mass spectrometry. J. Mass Spectrom.; 2005; 40:193-201).

The polypeptide of interest labeled with a stable isotope used as a standard in the method of the present invention may be produced recombinantly. Methods for the preparation of expression constructs and for the recombinant production of polypeptides and polypeptides are known in the art and are summarized in Ausubel, Current Protocols in Molecular Biology / Polypeptide science, Green Publishing Associates and Wiley Interscience, N.Y.(1994).

The polypeptide of interest labeled with a stable isotope used as a standard in the method of the present invention may be produced by chemical synthesis. Methods for the synthetic production of polypeptides or fragments thereof are known in the art, e.g. solid phase synthesis of polypeptides, and are summarized in Ausubel, Current Protocols in Polypeptide science, Green Publishing Associates and Wiley Interscience, N.Y.(1994). Solid phase peptide synthesis is the most common method used to prepare the synthetic peptides, and successful syntheses have been obtained using both Fmoc (9-fluorenylmethyloxycarbonyl) (Burdick, D; et al., J Biol Chem 1992, 267, 546-554) and Boc (t-butyloxycarbonyl)(Barrow, C. J.; et al., JMol Biol 1992, 225,1075-1093) methods for alpha-amino protection.

After addition of the polypeptide of interest standard, total polypeptide of interest comprising labeled and unlabeled polypeptides of interest may be isolated from body fluid, preferably from serum or CSF, by polypeptide chemical methods comprising immunoprecipitation and immunoaffinity chromatography.

Therefore, in a further embodiment, the polypeptide of interest in step (c) is isolated from body fluid by methods comprising polypeptide chemistry and immunochemistry.

For the determination of the content of polypeptide of interest of a source of polypeptide of interest containing aggregated polypeptide of interest, the aggregated polypeptide of interest has to be dissolved. In the method of the present invention, the aggregated polypeptide of interest is dissolved by methods comprising dissolution with solubilizing agents, and optionally mechanical solubilisation in the presence of the labeled polypeptide of interest standard. The solubilizing agents of the present invention may be all agents which have the capacity to dissolve aggregated polypeptide of interest, e.g. hexafluoropropanol, acid such as e.g. formic acid, urea-SDS. The mechanical solubilisation may comprise sonication. The dissolution procedure of the aggregated polypeptide of interest takes place in the presence of the added labeled polypeptide of interest standard thereby guaranteeing equal losses of the polypeptide of interest standard and the polypeptide of interest to be quantified.

Therefore, in a further embodiment, the polypeptide of interest in step (c) is isolated from polypeptide of interest deposits by methods comprising dissolution with solubilizing agents and optionally by sonication.

The isolated polypeptide of interest is subsequently prepared for analysis by mass spectrometry. The preparation for the analysis by mass spectrometry comprises methods which lead to an amelioration of the ionization of the Aβ to be analyzed. Methods leading to an amelioration of ionization comprise fragmentation by methods comprising chemical fragmentation and enzymatic digestion.

Subsequent the optionally fragmented polypeptide are bound with a chemical reactions to flight modifier. Chemical reactions with flight modifier comprise the charge derivatization of the peptides' free N-termini in order to enhance sensitivity and promote the formation of fragment ions by post-source decay MALDI mass spectrometry (J. Stults et al. (1993) Anal. Chem. 65,1703-1708; B. Spengler et al. (1997) Int. J. Mass Spectrom. 169-170, 127-140; Z. Huang et al. (1999) Anal. Biochem. 268, 305-317; Staudenmann W. and James P. in Proteome Research: Mass Spectrometry (P. James, Ed) Springer Verlag, Berlin (2001) 143-166). The isolated polypeptide may be directly reacted with flight modifier for preparation for analysis by mass spectrometry. Alternatively, the isolated polypeptide may be reacted with flight modifier after fragmentation. Chemical fragmentation may be carried out by the use of cyanogen bromide. The enzymatic digestion may be carried out with a protease selected from the group comprising endopolypeptidease Lys-C, trypsin, endopolypeptidease Glu-C and pepsin. In the method of the present invention, the isolated polypeptide may be dried and redissolved in a buffer before digestion with a protease. The fragmentation of the dissolved polypeptide may lead to a better limit of detection in the mass spectrometrical analysis, (Gruenigner et al. (2000). Identification of β-secretase-like activity using a mass spectrometry-based assay system. Nature biotechnology 18: 66-70).

Therefore, in a further embodiment, the isolated polypeptide of interest in step (d) is prepared for analysis by mass spectrometry by methods comprising chemical fragmentation and enzymatic digestion.

Before mass spectrometrical analysis, the dissolved and optionally fragmented polypeptide of interest may be desalted. Desalting of the sample (e.g., by ZipTip) is recommended when ionic compounds are present as competitors of ionization (e.g. introduced by buffers). These ions may completely suppress the signals of interest.

The isolated and optionally fragmented polypeptide of interest is then analyzed by mass spectrometry. Ionization techniques used for biological materials today comprise ESI (electrospray-ionization) and MALDI (matrix assisted laser desorption ionization). Preferably, the mass spectrometrical analysis used is a MALDI-TOF (time of flight) mass spectrometrical analysis. The spectrum of a MALDI-TOF-MS analysis consists primarily of the intact, singly charged molecule ions. Larger molecules, like polypeptides, may also yield multiply charged ions and, depending on their concentrations, singly charged multimers.

For Aβ as polypeptide of interest, the peak pattern of the natural ¹⁴N Aβ is base-line separated from its artificial ¹⁵N homologue in mass-spectrometry, thereby allowing the discrimination of the natural and the standard polypeptide of interest in the mass spectra.

The amount of polypeptide of interest such as i.e. Aβ that was present in the source of aggregated polypeptide of interest may be determined by different approaches to the analysis of the mass spectra: a) by comparing the heights of the two dominant peaks of the labeled standard (for Aβ: ¹⁵N-labeled amyloid standard) and of the polypeptide of interest from the source of polypeptide of interest, b) by comparing the heights of all the peaks of the separated peak patterns, c) by comparing the areas under the two dominant peaks, or d) by comparing the sum of the areas under all the peaks of the two different peak patterns. With the defined and known amount of labeled polypeptide of interest standard added at the beginning of the procedure, the amount of polypeptide present in the source of polypeptide can then be calculated. In cases where the amount of the polypeptide such Aβ that is present in a three-dimensional amyloid deposit, e.g. in a plaque, has to be determined a correction factor has to be included in the calculation.

Alzheimer's disease is characterized by extracellular deposits of amyloid fibrils in the patients brain. Many different variants of Aβ are known to occur with either heterogeneity at the amino- as well as carboxy-terminus. Of particular interest is the heterogeneity at the carboxy-terminus, since the longer form with 42 amino-acids (Aβ1-42) is much more prone to aggregation than the shorter form containing 40 amino acids (Aβ1-40). Therefore, it is important to differentiate between the two length variants. The crucial part thereby is the C-terminal. However, of the three parts generated with endopeptidase Lys-D, the C-terminal peptide fragment is not detectable with the Mass Spectrometry. A method of quantifying amyloid deposition before death is needed both as a diagnostic tool in mild or clinically confusing cases as well as in monitoring the effectiveness of therapies targeted at preventing Aβ deposition.

Therefore, the present invention further provides a method for detecting and quantifying AβX-40 and/or AβX-42 comprising
(a) providing source of the amyloid peptide AβX-40 and/or AβX-42,
(b) adding a defined amount of beta amyloid peptide AβX-40 and/or AβX-42 labeled with a stable isotope,
(c) isolating beta amyloid AβX-40 and/or AβX-42 in the presence of the labeled beta amyloid,
(d) digesting the isolated beta amyloid AβX-40 and/or AβX-42 with a protease,
(e) modifying the fragments of beta amyloid AβX-40 and/or AβX-42 with the ionization modifier,
(f) analyzing the digested beta amyloid peptide fragments by mass spectrometry, and
(g) determining the amount of the beta-amyloid peptide AβX-40 and/or AβX-42 that was present in the source by determining the amount of the length variants of the C-terminal fragments.

Preferably, X is 1, 2, 3, 4, 5, 6, 7, 8, 9, 10, 11 (Aβ1-40, Aβ2-40, Aβ3-40, Aβ4-40, Aβ5-40, Aβ6-40, Aβ7-40, Aβ8-40, Aβ9-40, Aβ10-40 Aβ11-40, Aβ1-42, Aβ2-42, Aβ3-42, Aβ4-42, Aβ5-42, Aβ6-42, Aβ7-42, Aβ8-42, Aβ9-42, Aβ10-42 and Aβ11-42). More preferably, X is 1.

Preferred length variants of the C-terminal fragments of (g) are Aβ29-40 and Aβ29-42. Also preferred length variants of the C-terminal fragments of (g) are Aβ11-40 and Aβ11-42.

These methods described above allow to differentiate between the two variants of the C-terminal fragment (AβX-40 and AβX-42) and to quantify the respective amounts of the variants in a sample, i.e. in a amyloid deposit obtained from tissue samples.

Another embodiment of the present invention is a method for the quantification of modified amyloid peptide comprising,
(a) providing source of the modified amyloid peptide Aβ,
(b) adding a defined amount of modified beta amyloid peptide Aβ labeled with a stable isotope,
(c) isolating the modified beta amyloid Aβ in the presence of the labeled beta amyloid,
(d) digesting the isolated modified beta amyloid Aβ with a protease,
(e) modifying the fragments of the modified beta amyloid Aβ with the ionization modifier,
(f) analyzing the digested modified beta amyloid peptide fragments by mass spectrometry, and
(g) determining the amount of modified beta-amyloid peptide Aβ that was present in the source.

The modification of Abeta peptide may be the cyclization of glutamate 11 of Aβ(11-16) thereby generating the N-terminal N-pyroglutamate specie Aβ(pyroGlu11-16). Preferably, the modification of the amyloid peptide is the oxidation of the side chain of methionine 35 to a methionine sulfoxide (Met-35(ox)). Methionine 35 means the amino acid methionine at the position 35 of the amino acid chain of the beta-amyloid peptide. The preferred source of the amyloid peptide are amyloid plaques in brains. The brains may derive from mammalian. The preferred brains are human brains or mouse brains.

Further forms of beta amyloid to be quantified by the methods of the present invention comprise cross-linked peptides, such as Aβ(X-38), Aβ(X-39), Aβ(X-40), Aβ(X-41), Aβ(X-42), Aβ(X-43), wherein X is 1, 2, 3, 4, 5, 6, 7, 8, 9, 10 or 11 and the peptide is pyroglutinated at position 3 and/or 11. These cross-linked peptides may optionally be fragmented by an array of proteolytic enzymes in order to fit the analytical window of the described method. The terms beta amyloid and Aβ are used equivalently in the present invention.

Preferred sources of beta amyloid or fragments thereof are amyloid deposits obtained from tissue samples, serum and CSF. Amyloid deposits obtained from tissue samples comprise dense (neuritic or senile) plaques, diffuse plaques, and amyloid deposits in small arterioles and venules, causing a microvascular angiopathy. The amyloid deposits mainly comprise aggregated beta amyloid, besides minor amounts of other components. Most preferred are amyloid plaques obtained from brain tissue.

Preferably, amyloid deposits are excised from tissue samples by laser dissection microscopy. Preferably, the amyloid deposits are excised from tissue slices, more preferably, they are excised from brain slices.

The beta amyloid may be present in the source of beta amyloid in aggregated or in soluble form. While Aβ in plaques is known to incorporate into amyloid fibrils, soluble nonfibrillar forms of Aβ do exist in vivo. Teller et al. (Teller, J. K.; et al., Nat Med 1996, 2, 93-95) detected soluble Aβ species in aqueous extracts of brains from Down's syndrome subjects and normal aged controls; the samples were obtained at autopsy from fetuses and from subjects ranging in age from 4 days to 61 years old. The amount of soluble Aβ was several-fold greater in the Down's syndrome subjects, and it increased with age. Furthermore, the elevation of soluble Aβ occurred well in advance of neuritic plaque formation. Kuo et al. (Kuo, Y. M.; et al., J Biol Chem 1996, 271, 4077-4081) examined aqueous extracts of brains from 8 AD subjects and 4 normal controls, and found a 6-fold increase in the amount of soluble Aβ. Ultrafiltration experiments on the soluble Aβ indicated the presence of Aβ oligomers.

The aggregated amyloid beta may be amyloid fibrils folding into "beta-pleated" sheet fibrils where amyloid fibrils are classified by the following criteria comprising (1) demonstration of Congo red binding and the display of green birefringence when viewed between crossed polarizers; (2) electron microscopic demonstration of fine nonbranching fibers, 6-10 nm in diameter; (3) presence of characteristic-structure; and (4) an x-ray fiber diffraction pattern resembling that of the cross- pattern seen in silk fibroin.

The presence of beta amyloid in the tissue sample or body fluid may be determined by methods comprising protein biochemistry, histochemistry and immunochemistry. Preferably, the presence of the aggregated beta amyloid in a tissue sample is determined by histochemical methods comprising staining with Congo Red or Thioflavin S, or by immunohistochemical methods. More preferably, the presence of the aggregated beta amyloid in a tissue sample is determined by double staining with histochemical and immunohistochemical methods. Most preferably, the presence of the aggregated beta amyloid in a tissue sample is determined by staining first with Congo Red followed by immunohistochemistry. Preferably, the presence of beta amlyoid in body fluid is determined by Western blotting of a body fluid sample.

The Aβ labeled with a stable isotope and added as a standard represents the same Aβ form as the one which is to be quantified in the source of Aβ. Therefore, the Aβ labeled with a stable isotope may be selected from the group comprising Aβ1-40, Aβ2-40, Aβ3-40, Aβ4-40, Aβ5-40, Aβ6-40, Aβ7-40, Aβ8-40, Aβ9-40, Aβ10-40 Aβ11-40, Aβ1-42, Aβ2-42, Aβ3-42, Aβ4-42, Aβ5-42, Aβ6-42, Aβ7-42, Aβ8-42, Aβ9-42, Aβ10-42 Aβ11-42 and Aβ29-X wherein X is 37, 38, 39, 40, 41, 42 or 43. The Abeta standard is labeled with at least one stable isotope selected from the group comprising ²H, ¹³C, ¹⁵N, and ¹⁸O. Preferably, the Abeta standard is labeled with ¹⁵N or ¹³C. More preferably, the Abeta standard is labeled with ¹⁵N. Preferably, the Abeta standard is labeled with as many stable isotopes as necessary for the separation of the isotope patterns in the mass spectra.

The Abeta standard labeled with a stable isotope is added in a defined amount. Preferably, the labeled Abeta standard is added in an amount in the same range as the effective amount of Abeta present in the source of polypeptide of interest. This amount may be determined in preliminary experiments.

The Abeta labeled with a stable isotope used as a standard in the method of the present invention may be produced by chemical synthesis. Methods for the synthetic production of polypeptides are known in the art, e.g. solid phase synthesis of polypeptides, and are summarized in Ausubel, Current Protocols in Polypeptide science, Green Publishing Associates and Wiley Interscience, N.Y.(1994). The demonstration that amyloid fibrils formed in vitro using synthetic Aβ peptides are identical to those isolated from senile plaques (Kirschner, D. A.; Inouye, H.; Duffy, L. K.; Sinclair, A.; Lind, M.; Selkoe, D. J. Proc Natl Acad Sci USA 1987, 84, 6953-6957) has validated the use of synthetic peptides in different studies. Solid phase peptide synthesis is the most common method used to prepare the synthetic peptides, and successful syntheses have been obtained using both Fmoc (9-fluorenylmethyloxycarbonyl) (Burdick, D; et al., J Biol Chem 1992, 267, 546-554) and Boc (t-butyloxycarbonyl)( Barrow, C. J.; et al., J Mol Biol 1992, 225, 1075-1093) methods for alpha-amino protection. While the Aβ peptides are moderately difficult to synthesize, standard coupling methods and side-chain protection strategies have proven to be sufficient for successful synthesis. For the introduction of stable isotopes into the Aβ standard, amino acids labeled with a stable isotope are used in the synthesis methods.

The Abeta amyloid peptide labeled with a stable isotope used as standard in the method of the present invention by be produce recombinantly. Methods for the recombinant production of natural Aβ are described in the art, e.g. in EP0641861. Preferably, the labeled Aβ may be produced by feeding recombinant *E coli* with ¹⁵N ammonium chloride. Other sources for stable isotopes comprise ¹³C-labeled glucose and extracts of algae grown on ¹⁵N-labeled substrates.

Having now generally described this invention, the same will become better understood by reference to the specific examples, which are included herein for purpose of illustration only and are not intended to be limiting unless otherwise specified, in connection with the following figures:

### Figures:

Figure 1 shows schematically the principle of the method of the present invention considering as example the identification of the C-terminal fragment of Abeta amyloid. Digestion of Abeta with endopeptidase Lys-D creates three fragments (Step 1)). The three fragments are treated with the flight modifier 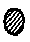 (Step 2)) which in this case binds specifically to the free amino group •. The bound flight modifier changes the flight behaviour of the fragments and enables the detection of the C-terminal fragment and the differentiation between Abeta(1-40) and Abeta (1-42).
Figure2 shows MALDI-TOF mass spectrogram (MS) obtained with 25 human dense plaques without ionization modifier (IM). The C-terminal fragments of the beta-amyloid peptide are not identifiable or quantifiable. It is not possible to distinguish between Abetal-40 and Abetal-42.
Figure 3 shows MALDI-TOF mass spectrogram obtained with 35 human dense plaques with ionization modifier (IM). Due to the IM the C-terminal fragments of Abeta are detectable and it is possible to distinguish between the Abetal-40 and Abetal-42.

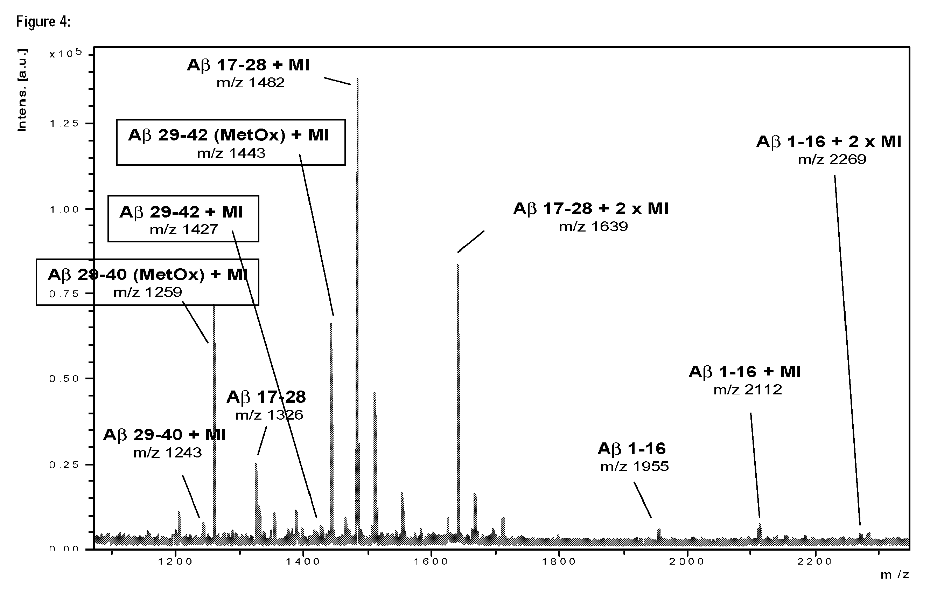

Figure 4 shows MALDI-TOF- mass spectrogram of 45 PS2APP transgenic mouse (Richards et al. (2003) PS2APP transgenic mice, coexpressing hAPPswe and hPS2mut, show age-related discrete brain amyloid deposition and inflammation associated with cognitive deficits. J Neurosci. 23, 8989) dense plaques with ionization modifier (IM). The IM allows to distinguish between the Abetal-40 and Abetal-42. Furthermore, minor modifications as the sulfoxidation of the methionine at position 35 may be detect by the method of the present invention.
Figure 5 shows Ion spray mass spectrogram from ammonia and 34 small amines (ethanolamine, glycine and cysteamin derivatized with the ionization modifier (IM)

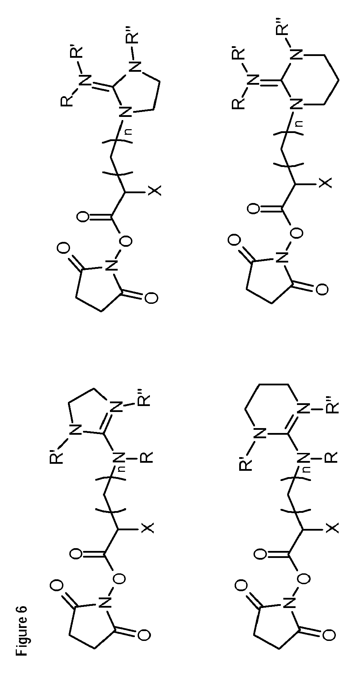

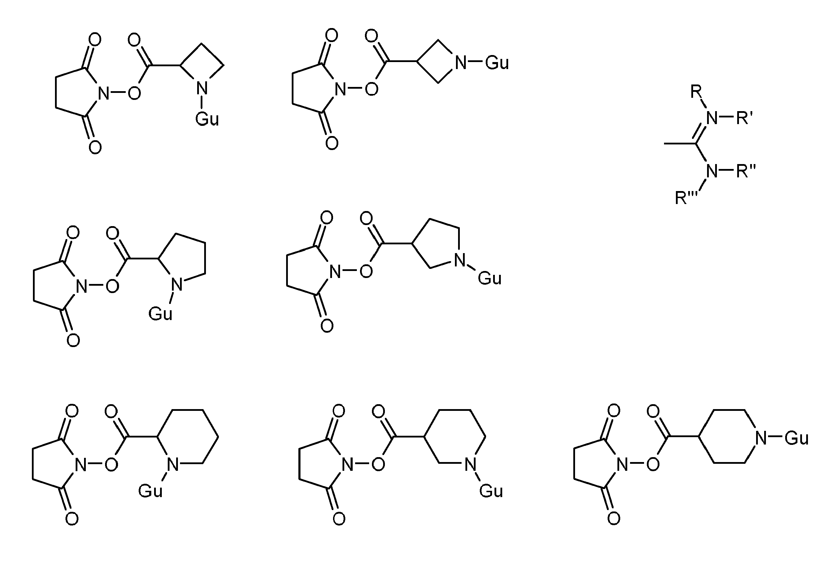

Figure 6 shows examples of the ionization modifier.

### EXAMPLES

Commercially available reagents referred to in the examples were used according to manufacturer's instructions unless otherwise indicated.

Example 1: Argininic acid-NHS adduct and its application in the detection of the carboxy-termini of Aβ in Alzheimer's disease plaques.

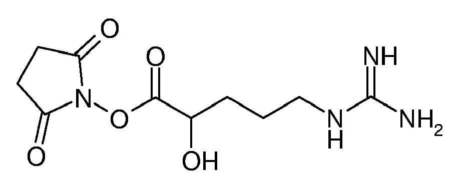

### Conversion of L-argininic acid into its HBF₄ salt

350 mg L-argininic acid (2 mMoles) are dissolved in 8 ml H₂O. This solution is titrated with ca 0.25 ml of 8 M tetrafluoroboric acid until the pH shows a drop from pH6 to pH2-3. After lyophilization, addition of 1 ml dimethyl formamide (DMF) and after a second lyophilization, a clear, very viscous oil is obtained.

### Synthesis of L-argininic acid N-hydroxy succinimide

25.5 mg O-(N-succinimidyl)-N,N,N',N'-tetramethyluronium tetrafluoroborate is dissolved in 0.32 ml DMF. To this solution, 70.6 mg of the L-argininic acid HBF₄ salt preparation and 60 µl pyridine are added. This mixture is incubated at 20 °C and the reaction is monitored by electron spray mass spectrometry (type: API 150 MCA). L-argininic acid N-hydroxy succinimide is very reactive and a substantial amount is already converted back to the free acid or to the amide (due to the ammonium ions contained in the buffer system used for mass spectrometry) during the few seconds required to dilute the sample and inject it. The proportion of the desired product is optimal after 5 hours. This preparation is termed crude ionization modifier.

### Reaction with fragmented Aβ peptides obtained from brain slices

This procedure is an extension of an already described procedure (Patent application EP 03024739.9 and Rüfenacht et al. 2005). According to this description, the plaques are excised from brain thin sections, stained with anti-Aβ antibodies or with Congo red, excised by laser dissection under the microscope and harvested in an eppendorf tube by laser pressure catapulting. Next, the aggregated Aβ contained in the plaques is dissolved with 70% formic acid, dried under vacuum and digested with endoproteinase Lys-C.

Typically, the volume of the digest is 10 µl, buffered with 10 mM NaHCO₃. To this digest 5 µl of crude ionization modifier solution is added and incubated for 1 h at 20 °C.

After this derivatization process, the procedure is the same as described in (Rüfenacht et al. 2005), involving first desalting with ZipTip, mixing with matrix in order to perform MALDI - TOF MS.

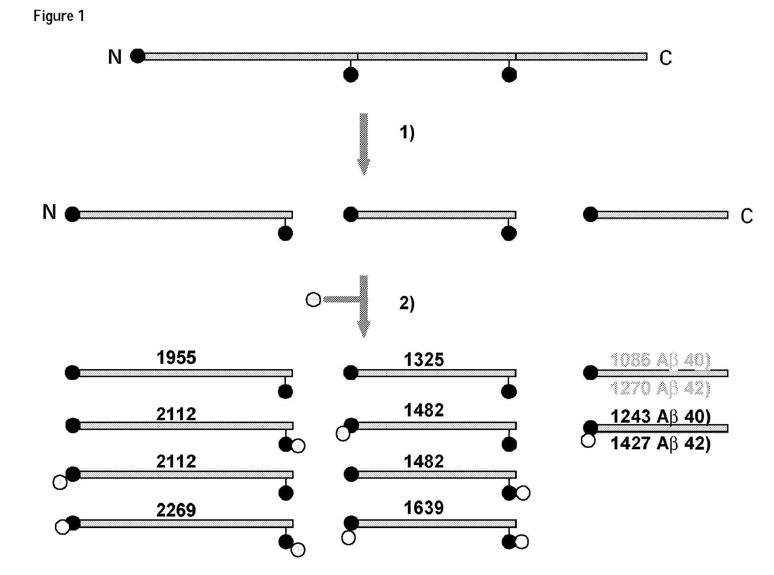

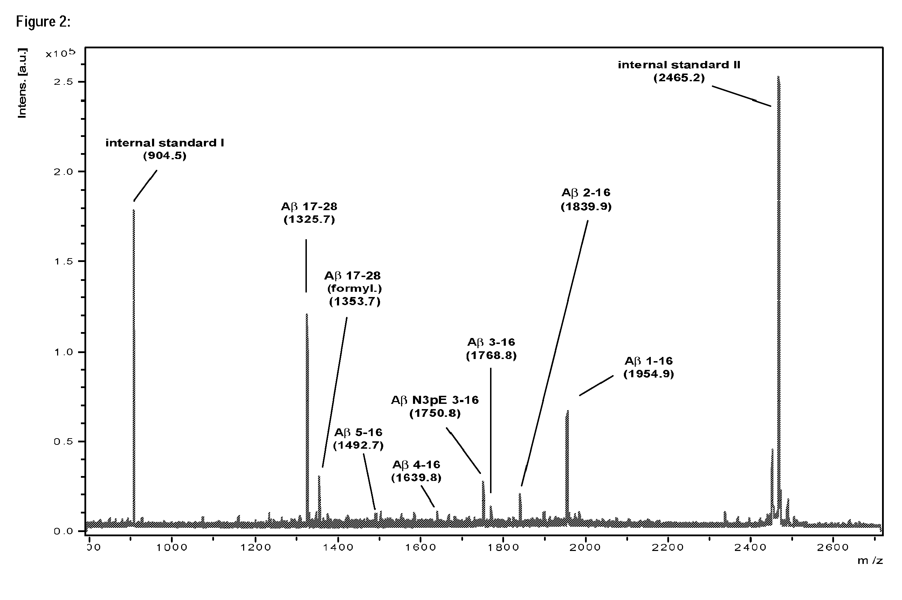

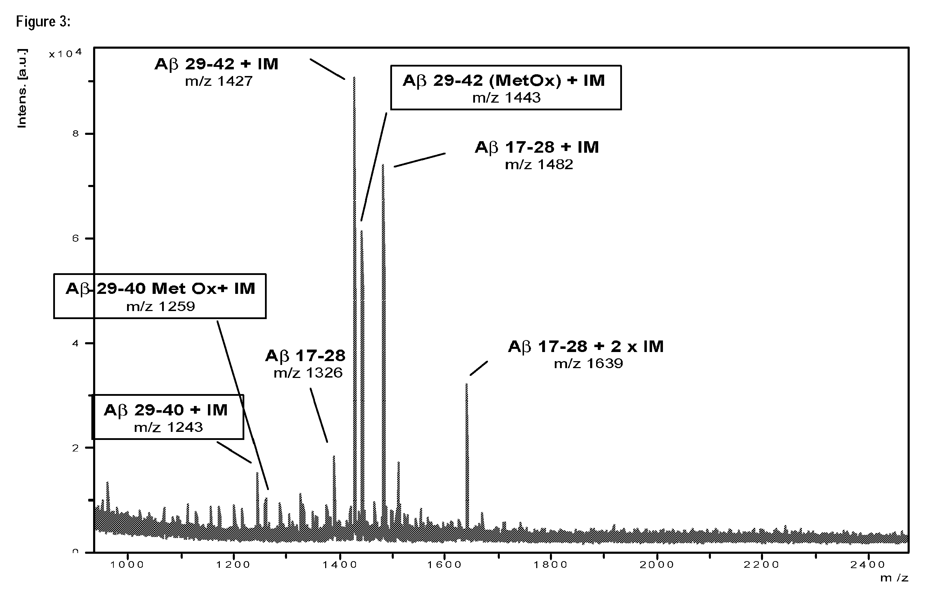

Optionally, a known amount of ¹⁵N-labelled Aβ can be spiked just before the dissolution of the fibres with 70% formic acid. This allows the quantification of the amount of the Aβ contained in the plaques. Natural ¹⁴N Aβ and recombinantly produced ¹⁵N Aβ are chemically identical, thus behave identical in respect to loss due to adsorption and in respect to protease cleavage and ionization. But when analyzed by mass spectrometry, the peaks deriving from the ¹⁵N Aβ are shifted to higher masses. By determining the proportions between the corresponding ¹⁴N and ¹⁵N peaks, the amount of endogenous Aβ can be determined. When the emphasis is on the carboxy-terminus one has to choose an adequate Aβ spike. In the tissue samples analyzed so far, we detected four different carboxy-termini in different proportions, depending from which origin the brain samples derived: Aβ(29-40) WT, Aβ(29-40) M35Mₒₓ, Aβ(29-42) WT and Aβ(29-42) M35Mₒₓ. WT stands for wild type having a methionine residue at position 35, whereas M35Mₒₓ stands for the oxidized form. For the quantification of the M35Mₒₓ forms one can use the ¹⁵N Aβ(1-40) or ¹⁵N Aβ(1-42) or any other ¹⁵N Aβ(X-Y) version (Riek et al. NMR studies in aqueous solution fail to identify significant conformational differences between the monomeric forms of two Alzheimer peptides with widely different plaque-competence, Aβ(1-40)ox and Aβ(1-42)ox; Eur. J. Biochem. 268, 5930-5936 (2001)). The CNBr cleavage procedure generates the methionine sulfoxide (Döbeli et al. A biotechnological method provides access to aggregation competent monomeric Alzheimer's 1-42 residue amyloid peptide; Bio Technology 13, 988-993 (1995)). To quantify the WT versions, one can use the recombinant fusion proteins. Digestion with endoproteinase Lys-C generates the authentic fragments Aβ(17-28) and Aβ(29-X). Examples are given in figures 1, 2 and 3.

Example 2: Synthesis of the 6-guanidohexanoic acid-NHS adduct and shift of the analyte peak into the analytical window of the mass spectrometer.

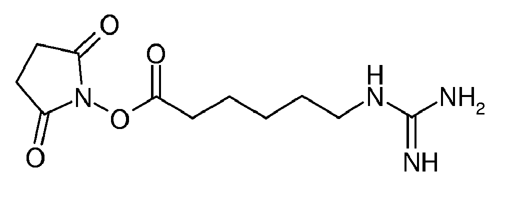

### Conversion of 6-guanidohexanoic acid into its HBF₄ salt

6-guanidohexanoic has a poor solubility in organic solvents, but when converted into its HBF₄ salt, it is soluble in methanol. 346 mg of 6-guanidohexanoic acid (2 mMoles) is dissolved in 0.25 ml H₂O and then neutralized with ca 250 µl 8 M tetrafluoroboric acid. After lyophilization, 463 mg of a white powder is obtained. Theoretical yield: 466 mg.

### Synthesis of the 6-guanidohexanoic acid ― N-hydroxysuccinimide adduct

6.3 mg 6-guanidohexanoic acid HBF₄ salt is dissolved in 120 µl methanol (200 mM) and 2.8 mg N-hydroxysuccinimide dissolved in 120 µl (200 mM) dimethylformamide are added to 70 mg N-cyclohexylcarbodiimide, N'-methyl polystyrene beads (Novo Biochem, Läufelfingen, Switzerland). The slurry is gently agitated on a rotary shaker at 20 °C and the reaction is monitored by electron spray mass spectrometry. After 8 hours the reaction is almost completed and can then be introduced into the desired assay for up to 48 hours.

Derivatization of small primary amines with the 6-guanidohexanoic acid ― N-hydroxysuccinimide adduct in order to shift the m/z values into the analytical window of an electron spray mass spectrometer

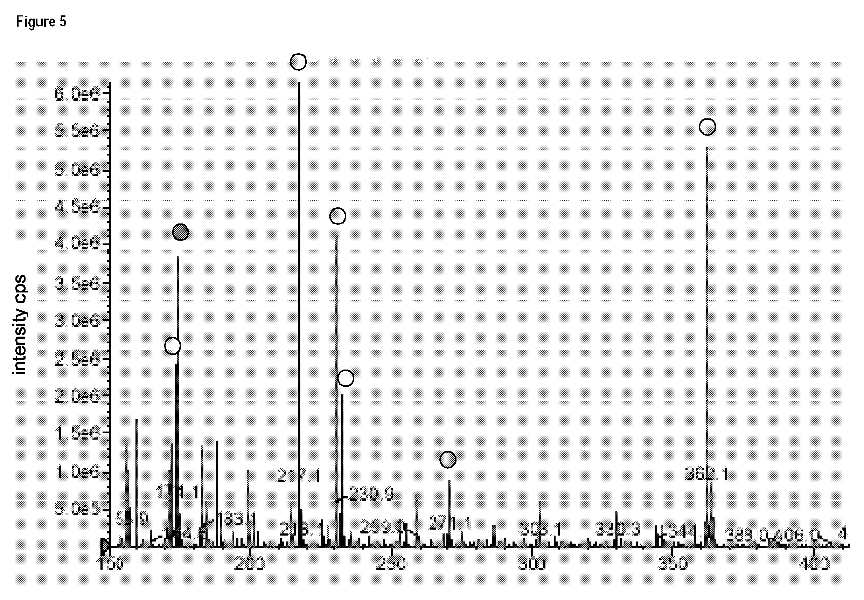

10 µl of a mixture containing ammonium chloride, ethanolamine, glycine and cysteamin (20 mM each) are treated with 10 µl of the 6-guanidohexanoic acid ― N-hydroxysuccinimide adduct (approximate concentration is 100 mM) for 1 hour at 20 °C. Prior to MS analysis, 30 µl water are added, then 5 µl are diluted with 500 µl MS buffer (50% acetonitrile and 50% 10 mM ammonium acetate) and 2 µl of this is injected into an electron spray mass spectrometer (type: API 150 MCA). The m/z values of the not derivatized amines would be 18 (NH₄ ion), 62 (ethanolamine ion), 76 (glycine ion) and 77 (cysteamine ion) and thus out of the analytical window of the mass spectrometer. As shown in figure 5 all the expected peaks of the modified amines are visible when conjugated to the ionization modifier.

## Claims

1. A compound of the general formula: 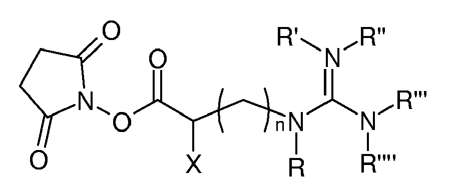 whereby
n = 0,1,2,3,4,5, 6, 7 or 8;
X = H, OH, F, Cl, Br, CH₃, C₂H₅, C₃H₇ or C₄H₉;
R = no residue, H, CH₃, C₂H₅, C₃H₇ or C₄H₉;
R' = no residue, H, CH₃, C₂H₅, C₃H₇ or C₄H₉;
R" = H, CH₃, C₂H₅, C₃H₇ or C₄H₉;
R''' = H, CH₃, C₂H₅, C₃H₇ or C₄H₉;
R'''' = H, CH₃, C₂H₅, C₃H₇ or C₄H₉;
or R' and R" or R" and R''' or R''' and R"" or R and R"" are bonded to each other to form a ring together with the nitrogen atoms to which they are attached, and R' and R" or R" and R''' or R''' and R"" or R and R"" together are:
-CH₂-(CH₂)ₚ-,
wherein p is 1, 2 or 3;
or X and R are bonded to each other to form a ring together with the nitrogen and carbon atoms respectively to which they are attached, and X and R together are:
- (CH₂)ₛ-.
wherein s is 1, 2, 3 or 4

2. The compound according to claim 1 wherein
n = 1
X = OH or H
R = H
R' = no residue
R" = H
R''' = H
R"" = H

3. The compound according to claim 1 wherein
n = 4
X = H
R = H
R' = no residue
R'' = H
R''' = H
R'''' = H

4. The compound according to anyone of the claim 1 to 3 as ionization modifier.

5. A method for the quantification of a polypeptide of interest from a source comprising said polypeptide of interest in labeled and unlabeled form, said method comprising the following steps:
(a) modifying the isolated polypeptide with a ionization modifier,
(b) analysing the prepared polypeptide by mass spectrometry, and thereby determining the amount of polypeptide of interest that was present in the source of polypeptide.

6. The method according to claim 5, wherein the ionization modifier is the compound according to any of the claims 1 to 3.

7. The method according to anyone of the claims 5 to 6, wherein the polypeptide is the C-terminal fragment Abeta(29-40), Abeta(29-42) of beta amyloid peptide.

8. The method according to anyone of the claims 5 to 7, wherein the labeled polypeptide is labeled with at least one stable isotope.

9. The method according to claim 8, wherein the labeled polypeptide is labeled with a stable isotope selected from the group comprising ¹⁵N, ¹³C, ¹⁸O and ²H.

10. The method according to anyone of the claims 5 to 9, wherein the prepared polypeptide in step (e) is desalted before analysis by mass spectrometry.

11. The method according to anyone of the claims 5 to 10, wherein the prepared polypeptide in step (e) is analyzed by MALDI-TOF mass spectrometry.

12. A method for detecting and quantifying a polypeptide of interest comprising
(a) providing a source of the polypeptide,
(b) adding a defined amount of said polypeptide labeled with a stable isotope to the source of (a),
(c) isolating unlabeled and labeled polypeptide,
(d) preparing the isolated polypeptide for analysis by mass spectrometry,
(e) modifying the isolated polypeptide with an ionization modifier,
(f) analyzing the prepared polypeptide by mass spectrometry, and
(g) determining the amount of polypeptide that was present in the source of polypeptide.

13. The method according to claim 12, wherein the ionization modifier is the compound of any of the claims 1 to 3.

14. The method according to anyone of the claims 12 to 13, wherein the source of the polypeptide in step (a) are obtained from a tissue sample.

15. The method according to anyone of the claims 12 to 14, wherein the source of the polypeptide in step (a) is amyloid deposits obtained from a tissue sample

16. The method according to claim 15, wherein the amyloid deposits are obtained from a tissue sample by excision by laser dissection microscopy.

17. The method according to claim 15 or 16 wherein the tissue sample is derived from brain.

18. The method according to claim 17 wherein the brain is a human or a rodent brain.

19. The method according to anyone of claims 12 to 14, wherein the source of the polypeptide in step (a) is body fluid.

20. The method according to anyone of claims 12 to 19, wherein the labeled polypeptide added in step (b) is a polypeptide which is recombinantly produced and labeled with at least one stable isotope.

21. The method according to anyone of the claims 12 to 19, wherein the labeled polypeptide added in step (b) is a polypeptide which is synthetically produced and labeled with at least one stable isotope.

22. The method according to anyone of claims 12 to 21, wherein the polypeptide added in step (b) is labeled with a stable isotope selected from the group comprising ¹⁵N, ¹³C, ¹⁸O and ²H.

23. The method according to anyone of claims 12 to 13, 19 to 22, wherein the polypeptide in step (c) is isolated from body fluid by methods comprising polypeptide chemistry and immunochemistry.

24. The method according to anyone of claims 12 to 18, 20 to 22, wherein the polypeptide in step (c) is isolated from deposits by methods comprising dissolution with solubilizing agents.

25. The method according to anyone of claims 12 to 24, wherein the isolated polypeptide in step (d) is prepared for analysis by mass spectrometry by methods comprising chemical fragmentation and enzymatic digestion.

26. The method according to claim 25, wherein the isolated polypeptide in step (d) is prepared for analysis by mass spectrometry by enzymatic digestion with a protease selected from the group comprising endopolypeptidease Lys-C, trypsin, and endopolypeptidease Glu-C.

27. The method according to claims 12 to 26, wherein the prepared polypeptide or in step (f) is desalted before analysis by mass spectrometry.

28. The method according to claims 12 to 27, wherein the prepared polypeptide in step (f) is analysed by MALDI-TOF mass spectrometry.

29. The method according to any one of claims 12 to 28, wherein the polypeptide of interest is the length variant AβX-40 and/or AβX-42 wherein X is 1, 2, 3, 4, 5, 6, 7, 8, 9, 10 or 11.

30. The method according to claim 29 wherein determining the amount of the beta-amyloid peptide AβX-40 and/or AβX-42 that was present in the source of step (g) is done by determining the amount of the length variants of the C-terminal fragments.

31. The method according to claim 30 wherein the length variants of the C-terminal fragments determined in step (g) are Aβ29-40 and/or Aβ29-42.

32. The method according to claim 30 wherein the length variants of the C-terminal fragments determined in step (g) are Aβ11-40 and/or Aβ11-42.

33. The method according to claim 29, wherein X is 1.

34. The method according to any one of the claims 12 to 28, wherein the polypeptide of interest is the C-terminal fragment Abeta(29-40) or Abeta(29-42) of the beta amyloid peptide.

35. The method according to any one of claims 12 to 28, wherein the polypeptide of interest is a modified amyloid peptide Aβ.

36. The method according to claim 35 wherein the modification is the oxidation of methionine 35 of the amyloid peptide.

37. The method according to claim 35 wherein the modification is the cyclization of glutamate 11 of the amyloid peptide.

38. The method substantially as herein before described especially with reference to the foregoing examples.
